# EUROPEAN PATENT SPECIFICATION

(11) **EP 3 097 424 B1**
(45) Date of publication and mention of the grant of the patent: **12.12.2018**
(21) Application number: 15712795.2
(22) Date of filing: 21.01.2015
(51) Int. Cl.: G01N 33/68, G01N 33/53

(54) **AN IN VITRO METHOD OF DIAGNOSING PARKINSON'S DISEASE**
IN-VITRO-VERFAHREN ZUR DIAGNOSE DER PARKINSONKRANKHEIT
MÉTHODE IN VITRO DE DIAGNOSTIC DE LA MALADIE DE PARKINSON

(30) Priority: 22.01.2014 IT TO20140039
(43) Date of publication of application: 30.11.2016
(73) Proprietor: Università degli Studi di Brescia, 25121 Brescia (IT); Fondazione Ospedale San Camillo IRCCS, 30126 Lido di Venezia (IT)
(72) Inventor: PIZZI, Marina, I-25123 Brescia (IT); LANZILLOTTA, Annamaria, I-25123 Brescia (IT); SPANO, Pierfranco, I-30126 Venezia (IT); ANTONINI, Angelo, I-30126 Venezia (IT)
(74) Representative: Comoglio, Elena
(86) International application number: PCT/EP2015/000102
(87) International publication number: WO 2015/110261

(56) References cited:
- WO-A2-2009/128050
- C. BAIGUERA ET AL: "Late-onset Parkinsonism in NF B/c-Rel-deficient mice", BRAIN, vol. 135, no. 9, 1 September 2012 (2012-09-01), pages 2750-2765, XP055146923, ISSN: 0006-8950, DOI: 10.1093/brain/aws193
- SARNICO I ET AL: "Activation of NF-Î B p65/c-Rel dimer is associated with neuroprotection elicited by mGlu5 receptor agonists against MPP+ toxicity in SK-N-SH cells", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 115, no. 5, 19 December 2007 (2007-12-19), pages 669-676, XP019633907, ISSN: 1435-1463

## Description

The present invention relates to an in vitro method of diagnosing Parkinson's disease (PD).

Parkinson's disease is the most common motor disorder and affects about 1.2 million people in Europe, and the therapy and rehabilitative treatment of which represent a huge economical challenge.

The main pathological features of Parkinson's disease are the degeneration of dopaminergic neurons of the Substantia Nigrastriatum, aggregation of alpha- synuclein into pathological agglomerates typical of the disease, Lewy bodies, synaptic dysfunctions, and neuroinflammation.

NF-kB transcription factors are known to have an important role in the progress of neurodegenerative processes, in that they regulate both inflammation and neuronal death (Pizzi and Spano, 2006; Camandola and Mattson, 2007). The NF-kB family of factors consists of 5 different subunits (c-Rel, p65/RelA, p50, Rel B, p52) which combine to form transcriptionally active dimers. Dimers that include the c-Rel factor promote neuroprotective effects through transcription of anti-apoptosis genes, among which the MnSOD enzyme, the Bcl-xL factor (Pizzi et al., 2002, 2005, Sarnico et al, 2009), and the mitochondrial UCP4 protein (Ho et al., 2012) genes. More recently, a pathogenetic role of the c-Rel factor appeared in the nigro-striatal degeneration and in the onset of parkinsonism. The deficiency of the c-Rel factor, in c-Rel gene knockout mice, was shown to induce, during ageing, a loss of dopaminergic neurons in the Substantia Nigra, with neuroinflammation, alpha-synuclein and iron accumulation. The lack of the c-Rel factor also causes a Parkinson-type motor symptomatology, with stiffness, hypokinesia, bradykinesia, which responds positively to treatment with L-dopa and benserazide, a treatment considered as the "gold standard" in the therapy for human Parkinson's disease. Baiguera C et al., BRAIN, vol. 135, no. 9, 1 September 2012, pages 2750-2765, establishes a link between artificially-created c-Rel deficiency in knocked-out mice and Parkinson's disease.

Several authors suggest that blood mononuclear cells (peripheral blood mononuclear cells, PBMC) represent a dopaminergic cell model for studying PD- related dysfunctions, as these cells express neuronal dopamine transporter (DAT), tyrosine hydroxylase, the key enzyme in DA synthesis, and alpha- synuclein (Caronti et al., 1999, 2001 ; Kim et al., 2004). Many studies are underway to validate the diagnostic usefulness of these signals and refine the search for biomarkers that are more and more disease-specific and - sensitive (Fuchs et al, 2008; Foulds et al., 2011 ; Gorostidi et al., 2012; Martins-Branco et al., 2012).

However, so far there are no reliable biomarkers that are validated for Parkinson's disease available. Moreover, the diagnosis is difficult, since many of the symptoms associated with PD, in particular earlier non-motor symptoms related to autonomic disorders, most commonly constipation, sleep-wake disorders such as REM disorders, sensory disorders such as pain and hyposmia, neuropsychiatric disorders such as anxiety and depression as well as cognitive difficulties, are characteristic not only for PD, but for a range of neurological diseases. It is important to develop diagnostic means that may be used to establish a reliable diagnosis at an early stage, so available therapies and medicaments can be used to the fullest possible benefit of the patients.

Thus, one problem underlying the present invention is to provide a biomarker that is reliable for Parkinson's disease.

Another problem underlying the present invention is to provide a peripheral biomarker for Parkinson's disease, which allows to make a diagnosis and monitor the progress of the disease at a lower cost, in association with or in place of the expensive imaging techniques currently in use.

Another problem underlying the present invention is to provide a test that may be used to diagnose patients having a neurological disease associated with symptoms similar to those of PD, in particular non-motor symptoms including constipation, depression, REM behaviour disorder, pain, smell loss (hyposmia) and cognitive difficulties.

These and other problems are solved by the present invention that provides an *in vitro* method of diagnosing Parkinson's disease or of monitoring the progress of Parkinson's disease based on the use of a new peripheral marker of Parkinson's disease, the NF-kappaB/c-Rel factor, in the blood mononuclear cells (peripheral blood mononuclear cells, PBMC) or brain tissue cells. The in vitro method of the invention is as defined in appended claim 1.

In a preferred embodiment of the first aspect, the biological sample is a protein extract from blood mononuclear cells.

In another preferred embodiment of the first aspect, the biological sample is a protein extract from brain tissue cells.

In another preferred embodiment of the first aspect, the human patient is a subject suspected of being affected by Parkinson's disease.

In another preferred embodiment of the first aspect, the human patient is a subject affected by Parkinson's disease.

In another preferred embodiment of the first aspect, the immunoassay is an ELISA assay.

In another preferred embodiment, the c-Rel capture reagent is immobilized.

In another preferred embodiment, active c-Rel factor is detected by the use of a secondary antibody binding to c-Rel, preferably a labeled secondary antibody.

The studies carried out by the present inventors, which will be illustrated in detail in the following experimental section, actually demonstrate that the activity of the NF-kappaB/c-Rel transcriptional protein, previously unexplored in the brain of patients affected by neurodegenerative diseases, is significantly decreased in blood cells of Parkinson's patients, which makes the utility of NF-kappaB/c-Rel actually plausible as a diagnostically significant peripheral biomarker.

Thus, the present inventors provided the *in vitro* method of diagnosing Parkinson's disease or of monitoring the progression of Parkinson's disease in a human patient, as defined in appended claim 1.

In a preferred embodiment, the term "diagnosis" means both the detection of the disease in a subject suspected of having Parkinson's disease and the monitoring of the progress of the disease and/or the monitoring of the effectiveness of the therapy in a subject already diagnosed as having Parkinson's disease. Therefore, the term "patient" means, as the case may be, a subject suspected of having Parkinson's disease or a subject already diagnosed as having Parkinson's disease.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as on January 23, 2014 as evidenced by text books and scientific publications.

Therefore, the term "diagnosis" does preferably not imply that the diagnostic methods according to the present invention will be definitive and sufficient to finalize the diagnosis on the basis of a single test, let alone parameter, but may refer to a contribution to what is referred to as a "differential diagnosis", i. e. a systematic diagnostic procedure considering the likelihood of a range of possible conditions on the basis of a range of diagnostic parameters.

The term "diagnosis" may also refer to a method used to distinguish between two or more conditions associated with similar or identical symptoms.

The term "diagnosis" may also refer to a method used to choose the most promising treatment regime for a patient. In other words, the method may relate to selecting a treatment regimen for a subject. Such treatment may comprise the administration of drugs known to slow down the progression of Parkinson's disease.

The patient's biological sample can be obtained from any suitable tissue, such as for instance brain tissue or, preferably, blood, that, in healthy subjects, contains endogenous active c-Rel factor. Therefore, in a preferred embodiment, the activity of the c-Rel factor is measured in a protein extract from a sample of the patient's blood mononuclear cells or, alternatively, in a protein extract from a sample of brain tissue.

The measuring of the activity of the c-Rel factor (also designated as "NF- kappaB/c-Rel" in order to point out that it is part of a NF-kappaB dimer), can be carried out with any per se known methodology, such as for example by immunoassay. In a preferred embodiment, the immunoassay is selected from the group comprising immunodiffusion techniques, immunoelectrophoretic techniques, light scattering immunoassays, light scattering immunoassays, agglutination techniques, labeled immunoassays such as those from the group comprising radiolabeled immunoassay, enzyme immunoassays, chemiluminscence immunoassays, and immunofluorescence techniques. The person skilled in the art is familiar with these methods, which are also described in the state of the art, for example in Zane, H. D. (2001), Immunology - Theoretical & Practical Concepts in Laboratory Medicine, W. B. Saunders Company, in particular in Chapter 14.

In a more preferred embodiment, the immunoassay is an ELISA assay. Kits for assaying the activity of the c-Rel factor are commercially available. By way of a non-limiting example, the Trans-NF-KB kit (Active Motif, Carlsbad, CA, United States of America) may be mentioned, which is used in the experimental section of the present description. The person of skill in the art can use appropriately or else modify as the case may require the commercially available kits, as well as carry out an immunoassay using the required reagents without the help of a prepacked kit. Such reagents essentially are a kB oligonucleotide and primary and secondary anti-c-Rel antibodies, each of which is per se known and commercially available.

A kB oligonucleotide is an isolated DNA sequence that contains the consensus sequence for the kB site (5'-GGGACTTTCC-3') naturally occurring on human DNA (Parry and Mackman, 1994). An oligonucleotide containing a consensus sequence for the human kB site is used for performing the invention.

An ELISA assay that uses a kB oligonucleotide as the capture element, such as for example the above-mentioned Trans-NF-KB (Active Motif, Carlsbad, CA, United States of America), essentially relies on the binding activity of NF-kB dimers (containing active c-Rel factor), which are present in the biological sample to be tested, to the kB oligonucleotide immobilized on a solid support, for instance on the bottom of the wells of an ELISA plate. The active c-Rel factor within the NF-kB dimer is recognized by a specific anti-c-Rel antibody.

Evaluating the results of the assay usually involves comparing a set of data from at least one patient and a set of data from healthy subjects. The patient is suspected of suffering or of being likely to suffer from the neurological disease in the future if the amount of active c-Rel in his or her sample is considerably lower than the activity measured in samples from healthy subjects. For example, the activity in the patient's sample may be less than 40, preferably less than 50, 60, 70, 80, 90 or 95 % of the activity in samples from healthy subjects.

The progress of the disease and the effect of treatment on the course of the neurological disease may be monitored by repeatedly measuring the amount of active c-Rel factor over time.

The activity of a second constitutively active protein in the sample may be measured as a control to ensure proper handling and thus preservation of activity in the sample. The c-Rel factor activity may be determined as the ratio of c-Rel factor activity and control protein activity in the sample.

Also disclosed is a kit for carrying out the method of the invention. Such a kit may comprise instructions detailing how to use the kit and a means for contacting a c-Rel factor capture reagent, for example the kB oligonucleotide, with a sample from a subject, preferably a human, and means to detect active c-Rel factor, for example the primary and secondary anti-c-Rel antibodies. Furthermore, the kit may comprise a positive control, for example a batch of active c-Rel factor and a negative control, for example a protein having no detectable affinity to common c-Rel factor capture reagents. Finally, such a kit may comprise a stand- ard solution for preparing a calibration curve or reagents for preparing such a standard solution.

The following experimental section is provided solely by way of illustration. The scope of the invention is defined by the appended claims.

### Exprimental section

### Isolation of blood mononuclear cells (PBMC) from blood samples

PBMCs were collected from the venous blood of 23 PD-affected donors and 17 healthy subjects of both sexes and between 50 and 84 years of age.

PBMCs represent the cellular component of blood deprived of platelets, red blood cells and polymorphonucleated cells. They are made up of approximately 60%- 70% lymphocytes and the remaining 30-40% are monocytes.

The PBMC preparation was done on a HISTOPAQUE-1077 gradient (polysaccharose and sodium diatrizoate, density 1.077 ± 0.001 g/ml; Sigma Aldrich, St. Louis, MO, United States of America) according to the following procedures.

The HISTOPAQUE-1077 and phosphate buffer (PBS) solutions (NaCl 8g/l; KCl 0.2 g/l; Na₂HPO₄*2H₂O 1.44 g/l; KH₂PO₄ 0.24 g/l; pH 7.4) were adjusted to room temperature. Ten ml of blood were collected from the subjects into vacutainer tubes containing EDTA as an anticoagulant. All the collected blood was combined in a single 15 ml Falcon tube and centrifuged at 1200 x g (about 2000 rpm) for 10 minutes at room temperature. The blood cell pellet was adjusted to a volume of 14 ml with PBS. Two 15-ml Falcon tubes were prepared, each containing 6 ml of HISTOPAQUE-1077. 7 ml of diluted blood were layered onto the HISTOPAQUE-1077 layer in each Falcon tube, and then centrifuged at 400 x g for 30 minutes without breaks. The PBMC ring that separates above the HISTOPAQUE-1077 was collected and transferred into another 50-ml Falcon tube. The volume was adjusted to 40 ml with PBS, followed by centrifugation at 250 x g for 15 minutes. The supernatant was discarded and the cell pellet was resuspended. The volume was adjusted to 40 ml with PBS. This was again centrifuged at 250x g for 15 minutes, the supernatant was discarded, the pellet was resuspended for about 30 seconds in ddH2O to lyse the contaminating red blood cells. The final volume was adjusted to 40 ml with PBS and centrifuged at 250 x g for 15 minutes. The supernatant was discarded and the pellet was resuspended for cell counting with the Burker chamber. Known amounts of the PBMCs thus obtained were pelleted again by centrifuging at 250 x g for 15 min and stored at -80°C until analysis.

### Extraction of total proteins from Substantia Nigra

The frozen human substantia nigra from 10 Parkinson and 10 control cases were supplied by Parkinson's Uk Brain Bank. Approximately 30 mg of tissue was resuspended in 200 µI of cold Lysis buffer consisting of 50 mM Tris-HCl (pH 7.6), 150 mM NaCl, 1 mM Na₃VO₄, 10 mM NaF, 2 mM EDTA, 0.5% NP40, 1 mM PMSF and 1x protease inhibitor cocktail (Sigma Aldrich) and transferred into new 1.5-ml tubes, keeping them on ice. The tissues were lysed by sonication: three 5- second rounds, each at a power of 35 W, for each sample. This was then centrifuged at 15000 x g for 20 minutes at 4°C. The supernatant was collected in other eppendorf tubes, the proteins were weighed out and aliquots were prepared, and stored at -80°C.

### Extraction of total proteins from PBMC

The PBMC total proteins were prepared according to the following procedures.

The pellet from 10 ml of whole blood was resuspended in 100 µI of cold Lysis buffer consisting of 50 mM Tris-HCl (pH 7.6), 150 mM NaCl, 1 mM Na₃VO₄, 10 mM NaF, 2 mM EDTA, 0.5% NP40, 1 mM PMSF and 1x protease inhibitor cocktail (Sigma Aldrich) and transferred into new 1.5-ml tubes, keeping them on ice. The PBMCs were lysed by sonication: two 5-second rounds, each at a power of 35 W, for each sample. This was then centrifuged at 15000 x g for 20 minutes at 4°C. The supernatant was collected in other eppendorf tubes, the proteins were weighed out and aliquots were prepared, and stored at -20°C. 310 µg of total proteins are obtained from 10 ml of whole blood, extracted in 100 ml of Lysis buffer.

### Measuring out c-Rel factor activity by ELISA assay (Enzyme Linked Immunosorbent Assay)

The binding ability of the c-Rel factor, within a NF-kB dimer, to a consensus DNA sequence (oligonucleotide) for the kB site, naturally occurring on human DNA, was measured through an ELISA test, by using the Trans-NF-KB kit (Active Motif, Carlsbad, CA, United States of America).

The preparation of the protein extracts was performed according to the instructions for sample preparation provided by the firm producing the kit. The reagents and procedures used were included in the kit for assaying the c-Rel factor, with the exception of the anti-c-Rel antibody which was substituted in our procedure by the primary anti-c-Rel antibody (C) (# sc - 71 Santa Cruz Biotechnology).

The total extracts (50 pg) obtained from human PBMCs or from substantia nigra (25 ig) were measured out in 96-well plates containing the kB oligonucleotide immobilized at a high density. The active form of the c-Rel subunit, bound to the target DNA sequence, was detected using the Santa Cruz Biotechnology primary anti-c-Rel antibody (lot #D1107, 1 :50 was used in the PBMC analyses. Lot # H1312 1 :250 was used for substantia nigra analysis)) and then the secondary HRP-conjugated antibody (1 :1000) provided by the kit. The developing solution was added for 5 minutes and the absorbance of the sample was read with a spectrophotometer at a wavelength of 450 nm. The data obtained are expressed as the difference in absorbance observed in the presence of the total extract compared to that observed in the absence of the total extract (blank).

### Results

The activity of the c-Rel factor was measured as the ability of the protein present in the PBMC and substantia nigra protein extract to bind the oligonucleotide probe containing the kB sequence, immobilized onto the bottom of the ELISA kit cuvette. The c-Rel protein was then identified among the proteins bound to the oligonucleotide probe by a specific anti-human c-Rel antibody.

The amount of binding of c-Rel to the kB probe reflects the amount of NF-kB/c- Rel dimers that are found in the cells in the free form at the time of the preparation of the sample. Those dimers, once released from the NF-kB-lkB complex, during the activation process of the factor itself, are ready to translocate from the cytoplasm to the nucleus in order to bind to the kB sites of the DNA.

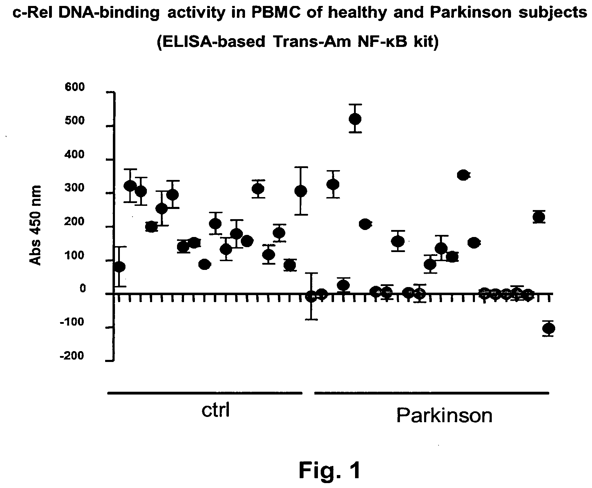

The analysis carried out in 23 Parkinson's patients and 17 controls revealed a significant correlation between the decrease in c-Rel activity and disease (Figure 1). The statistical analysis by Mann-Whitney U test for independent samples demonstrates a significant difference (p = 0.010) between the two examined groups.

The evaluation of c-Rel factor activity in the substantia nigra of 10 Parkinson's disease (PD) patients showed a significant reduction of activity in PD cases. The statistical analysis by Mann-Whitney U test for independent samples demonstrates a significant difference (p = 0.0433) between the two examined groups.

### Conclusions

The results outlined in Figure 1 demonstrate that the activity of the c-Rel factor is reduced compared to healthy individuals in the blood cells of a considerable number of subjects affected by Parkinson's disease. Therefore, the analysis of the c-Rel factor represents a peripheral biomarker useful to improve the diagnosis accuracy of Parkinson's disease in the initial clinical stage. Moreover, it is a marker of the therapy effectiveness. The low degree c-Rel factor activity in PBMCs represents an indicator of the degenerative process present within the brain areas affected by the disease. Similar data could be obtained if the c-Rel factor activity in the substantia nigra of PD patients was compared to samples from healthy subjects.

### Bibliography

Baenkler H. W. (2012), General aspects of autoimmune diagnostics, in Renz, H., Autoimmune diagnostics, 2012, de Gruyter
Camandola & Mattson, 2007, Expert Opin Ther Targets, 11 : 123-32.
Caronti, et al., 1999, Neuroreport 10, 2907-2910.
Caronti et al., 2001 , J Neural Transm 108: 803-807.
Foulds et al., 2011 , FASEB J.25(12):4127-37.
Fuchs J et al., 2008, FASEB J. 22:1327-34.
Gorostidi et al., 2012, PLOSone 7(12): e52312.
Ho et al., 2012, Free Radic Biol Med. 53(2):383-94.
Kim et al., 2004 FASEB J. 18: 1615-7.
Martins-Branco D et al., 2012, Exp Neurol.238(2):89-99.
Parry & Mackman, 1994 J Biol Chem. 269:20823-5.
Pizzi et al., 2002, J. Biol Chem. 277: 20717-23.
Pizzi et al., 2005, Cell Death Diff Cell Death Differ. 12(7):761-72.
Pizzi M, Spano P., 2006, Eur J Pharmacol 2006; 545: 22-8.
Sarnico et al., 2009, J Neurochem. 108: 475-85.

## Claims

1. An *in vitro* method of diagnosing Parkinson's disease in a human patient or of monitoring the progress of Parkinson's disease in a human patient, comprising measuring the amount of active c-Rel factor in a biological sample comprising endogenous c-Rel factor from the human patient, wherein the amount of active c-Rel factor is measured by an immunoassay which uses as capture agent an oligonucleotide containing a consensus sequence for the human kB (kappaB) site, a decrease in the active c-Rel factor in the biological sample from the human patient compared to a healthy control being indicative of the presence or progress of Parkinson's disease.

2. The in vitro method according to claim 1, wherein the consensus sequence for the human kB site is 5'-GGGACTTTCC-3'.

3. The *in vitro* method according to claim 1 or 2, wherein the biological sample comprises a protein extract from blood mononuclear cells.

4. The *in vitro* method according to claim 1 or 2, wherein the biological sample comprises a protein extract from brain tissue cells.

5. The *in vitro* method according to any one of claims 1 to 3, wherein the human patient is a subject suspected of being affected by Parkinson's disease.

6. The *in vitro* method according to any one of claims 1 to 3, wherein the human patient is a subject affected by Parkinson's disease.

7. The *in vitro* method according to any one of claims 1 to 6, wherein the immunoassay is an ELISA assay.

## Patentansprüche

1. *In* vitro-Verfahren zum Diagnostizieren der Parkinson-Krankheit bei einem menschlichen Patienten oder zur Überwachung des Fortschreitens der Parkinson-Krankheit bei einem menschlichen Patienten, aufweisend das Messen der Menge des aktiven c-Rel-Faktors in einer biologischen Probe, die den endogenen c-Rel-Faktor des menschlichen Patienten aufweist, wobei die Menge des aktiven c-Rel-Faktors durch einen Immunotest gemessen wird, welcher als Erfassungsmittel ein Oligonukleotid verwendet, das eine Konsensussequenz für die menschliche kB (kappaB)-Stelle enthält, wobei eine Abnahme des aktiven c-Rel-Faktors in der biologischen Probe des menschlichen Patienten gegenüber einer gesunden Kontrollprobe auf das Vorhandensein oder den Verlauf der Parkinson-Krankheit hinweist.

2. *In* vitro-Verfahren nach Anspruch 1, wobei die Konsensussequenz für die menschliche kB-Stelle 5'-GGGACTTTCC-3' ist.

3. *In* vitro-Verfahren nach Anspruch 1 oder 2, wobei die biologische Probe ein Proteinextrakt aus mononukleären Blutzellen umfasst.

4. *In* vitro-Verfahren nach Anspruch 1 oder 2, wobei die biologische Probe ein Proteinextrakt aus Hirngewebezellen umfasst.

5. *In* vitro-Verfahren nach einem der Ansprüche 1 bis 3, wobei der menschliche Patient ein Subjekt ist, bei dem der Verdacht besteht, das es von der Parkinson-Krankheit betroffen ist.

6. In vitro-Verfahren nach einem der Ansprüche 1 bis 3, wobei der menschliche Patient ein von der Parkinson-Krankheit betroffenes Subjekt ist.

7. In vitro-Verfahren nach einem der Ansprüche 1 bis 6, wobei der Immunotest ein ELISA-Test ist.

## Revendications

1. Méthode *in vitro* de diagnostic de la maladie de Parkinson chez un patient humain ou de surveillance de l'évolution de la maladie de Parkinson chez un patient humain, comprenant une mesure de la quantité de facteur c-Rel actif dans un échantillon biologique comprenant un facteur c-Rel endogène du patient humain, dans lequel la quantité de facteur c-Rel actif est mesurée par un essai immunologique qui utilise comme agent de capture un oligonucléotide contenant une séquence consensus du site kB humain (kappa B), une diminution du facteur c-Rel actif dans l'échantillon biologique du patient humain par rapport à un témoin sain étant indicatif de la présence ou de l'évolution de la maladie de Parkinson.

2. Méthode *in vitro* selon la revendication 1, dans laquelle la séquence consensus pour le site kB humain est 5'-GGGACTTTCC-3'.

3. Méthode *in vitro* selon la revendication 1 ou 2, dans laquelle l'échantillon biologique comprend un extrait de protéine de cellules sanguines mononucléaires.

4. Méthode *in vitro* selon la revendication 1 ou 2, dans laquelle l'échantillon biologique comprend un extrait de protéine de cellules de tissu cérébral.

5. Méthode *in vitro* selon l'une quelconque des revendications 1 à 3, dans laquelle le patient humain est un sujet suspecté d'être affecté par la maladie de Parkinson.

6. Méthode *in vitro* selon l'une quelconque des revendications 1 à 3, dans laquelle le patient humain est un sujet affecté par la maladie de Parkinson.

7. Méthode *in vitro* selon l'une quelconque des revendications 1 à 6, dans laquelle l'essai immunologique est un essai ELISA.
